# EUROPÄISCHE PATENTSCHRIFT

(11) **EP 0 925 373 B1**
(45) Veröffentlichungstag und Bekanntmachung des Hinweises auf die Patenterteilung: **22.10.2003**
(21) Anmeldenummer: 97938922.8
(22) Anmeldetag: 18.08.1997
(51) Int. Cl.: C12Q 1/68

(54) **VERFAHREN ZUM NACHWEIS VON NUKLEINSÄUREN UNTER ERMITTLUNG DER MASSE**
PROCESS FOR DETECTING NUCLEIC ACIDS BY MASS DETERMINATION
PROCEDE POUR LA DETECTION D'ACIDES NUCLEIQUES PAR DETERMINATION DE LA MASSE

(30) Priorität: 20.08.1996 DE 19633436
(43) Veröffentlichungstag der Anmeldung: 30.06.1999
(73) Patentinhaber: PNA Diagnostics A/S, 2600 Glostrup (DK)
(72) Erfinder: BERGMANN, Frank, D-82393 Iffeldorf (DE); HERRMANN, Rupert, D-82362 Weilheim (DE); KOBOLD, Uwe, D-82407 Wielenbach (DE)
(74) Vertreter: Christiansen, Ejvind
(86) Internationale Anmeldenummer: EP9704494
(87) Internationale Veröffentlichungsnummer: WO98007885

(56) Entgegenhaltungen:
- SAMBROOK J. ET AL.,: "Molecular cloning: A laboratory manual" 1989 , COLD SPRING HARBOR LABORATORY , COLD SPRING HARBOR, N.Y. XP002049608 siehe Seite 7.58 - Seite 7.65
- TANG K ET AL: "DETECTION OF 500-NUCLEOTIDE DNA BY LASER DESORPTION MASS SPECTROMETRY" RAPID COMMUNICATIONS IN MASS SPECTROMETRY, Bd. 8, Nr. 9, September 1994, Seiten 727-730, XP000608100

### SEQUENZPROTOKOLL

### (1) ALLGEMEINE ANGABEN:

(i) ANMELDER:
   (A) NAME: Boehringer Mannheim GmbH
   (B) STRASSE: Sandhoferstr. 116
   (C) ORT: Mannheim
   (E) LAND: DE
   (F) POSTLEITZAHL: 68305
   (G) TELEFON: 0621 759 4348
   (H) TELEFAX: 0621 759 4457
(ii) BEZEICHNUNG DER ERFINDUNG: Verfahren zum Nachweis von Nukleinsäuren unter Ermittlung der Masse
(iii) ANZAHL DER SEQUENZEN: 1
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)

### (2) ANGABEN ZU SEQ ID NO: 1:

(i) SEQUENZKENNZEICHEN:
   (A) LÄNGE: 45 Basenpaare
   (B) ART: Nucleotid
   (C) STRANGFORM: Einzelstrang
   (D) TOPOLOGIE: linear
(ii) ART DES MOLEKÜLS: Sonstige Nucleinsäure
   (A) BESCHREIBUNG: /desc = "Oligodeoxyribonukleotid"
(iii) HYPOTHETISCH: NEIN
(xi) SEQUENZBESCHREIBUNG: SEQ ID NO: 1:
   TATTAGGCCA TCGTCTTCAT GGTCCAGAAC ACCAACAGGA AGACC 45

## Beschreibung

Gegenstand der Erfindung ist ein Verfahren zum Nachweis von Nukleinsäuren aufgrund ihrer Masse sowie ein zur Durchführung dieses Verfahrens geeignetes Reagenz.

Nukleinsäuren werden in der Analytik immer mehr als Anzeichen für die Anwesenheit von Mikroorganismen oder für den genetischen Zustand eines Organismus herangezogen. Dies ist insbesondere begründet durch die Menge an Informationen, die in Nukleinsäuren enthalten ist. Insbesondere auf dem Gebiet der Diagnostik von genetischen Erkrankungen wurde der Nachweis von Mutationen auf Nukleinsäureniveau als sinnvoll erachtet.

In einer Art von Nukleinsäurenachweisen wird die Hybridisierung von markierten Sonden mit den nachzuweisenden Nukleinsäuren als Zeichen fiir die Anwesenheit der nachzuweisenden Nukleinsäure ausgewertet. Diese Nachweise können sowohl heterogen als auch homogen durchgeführt werden. In heterogenen Assays wird nach einem Probenvorbereitungsschritt die isolierte Targetnukleinsäure meist durch ein Nukleinsäurevermehrungsverfahren, wie z. B. die PCR gemäß EP-B-0 200 362, amplifiziert, dann an eine Festphase immobilisiert und anschließend mit einer analytspezifischen, direkt oder indirekt mit einer signalgebenden Reportergruppe markierten Sonde detektiert. Häufig verwendete Markierungen sind radioaktiver oder nicht-radioaktiver Natur. Als Markierung kommen auch Enzyme in Frage, wobei das Enzym eine bestimmte Nachweisreaktion katalysiert. In homogenen Assays wird keine Bindung an eine Festphase durchgeführt. Hierfür werden oft Interkalatorverbindungen eingesetzt, die nach Interkalation in den Doppelstrang aus Analytnukleinsäure und Sonde eine Änderung einer physikalischen Meßgröße erfahren, z. B. Verstärkung ihrer Fluoreszenz (Nucleic Acids Research, 1995, 23 (5), 753 - 760) oder Änderung der Fluoreszenzpolarisation. In homogenen Assays nach WO 92/02638 wird eine Sonde, die sowohl ein Fluoreszenzlabel als auch einen Fluoreszenzquencher enthält, eingesetzt. Im Verlaufe des Assays wird das Label vom Quencher abgespalten und die Fluoreszenzaktivität detektiert. Die vorgenannten heterogenen und homogenen Assays setzen den Einsatz chemisch aufwendiger, speziell markierter Sonden voraus. Diese sind einerseits aufwendig in ihrer Herstellung und andererseits muß bei jedem Label festgestellt werden, ob es nicht die Hybridisierungseffizienz nachteilig beeinflußt.

In US-A-5,453,247 ist ein Verfahren zur Sequenzierung von DNA beschrieben. Hierzu wird auf der Basis der DNA, deren Nukleotidsequenz bestimmt werden soll, ein Set von Nukleinsäurefragmenten erzeugt, wobei jedes Set mit einer oder mehr der vier Nukleobasen endet. Es müssen alle möglichen Fragmente (einzelsträngig) des Bereiches erzeugt werden, der sequenziert werden soll. Die einzelnen Fragmente unterscheiden sich jeweils um ein Nukleotid. Die Masse jedes dieser Fragmente wird massenspektrometrisch bestimmt.

In WO 95/15974 ist ein Verfahren zum Nachweis von Nukleinsäuren beschrieben, bei dem eine markierte Nukleinsäureanalogensonde mit der nachzuweisenden Nukleinsäure in Kontakt gebracht wird, die Nukleinsäure bis auf den durch die Sonde geschützten Bereich abgebaut und die Anwesenheit des Komplexes mit Hilfe der Markierung an der Sonde nachgewiesen wird.

In J. Am. Chem. Soc. 1995, 117, 831 - 832 und Rapid Communications in Mass Spectrometry, 10, 47-50 (1996), sind Massenspektren von Komplexen aus einer Peptidnukleinsäure (PNA) und einem Oligonukleotid beschrieben, in denen das Oligonukleotid synthetisch hergestellt wurde und gleich groß oder größer ist als der Bereich, der durch Basen-Basen-Wechselwirkungen rnit dem PNA-Molekül Bindungen eingeht.

Aufgabe der vorliegenden Erfindung war es, ein Nukleinsäurenachweisverfahren bereitzustellen, welches ohne markierte Sonden auskommt und das eine parallele Bestimmung von unterschiedlichen Nukleinsäuren zuläßt.

Gegenstand der Erfindung ist daher ein Verfahren zum Nachweis von Nukleinsauren durch Bindung einer Sonde P an eine in der Nukleinsäure enthaltene Teilsequenz S unter Erzeugung eines Bindeproduktes B1, Abbau der Nukleinsäure unter Erzeugung eines eine Teilnukleinsäure definierte Länge enthaltenden Bindeprodukts B2 und Nachweis des Bindeprodukts B2 oder eines Teils davon aufgrund seiner Masse.

Unter einer Nukleinsäure im Sinne der Erfindung können alle dem Fachmann bekannten Nukleinsäuren verstanden werden, z. B. RNA und DNA. Sowohl doppelsträngige als auch einzelsträngige Nukleinsäuren können nachgewiesen werden. Da die Sonde P strangverdrängende Eigenschaften hat, ist es möglich, doppelsträngige Nukleinsäuren ohne vorherige Denaturierung in das erfindungsgemäße Verfahren einzubringen.

Die nachzuweisenden Nukleinsäuren sind definiert abbaubar. Die Quelle der nachzuweisenden Nukleinsäuren ist nicht wesentlich. Bevorzugt wird eine die nachzuweisende Nukleinsäure enthalteade Probe jedoch einer Vorbehandlung unterzogen, so daß die Nukleinsäuren in Lösung und für gelöste Sonden zugänglich vorliegen. Insbesondere bei Nachweisen von Nukleinsäuren aus Zellen oder Organismen hat es sich als vorteilhaft erwiesen, die Nukleinsäuren durch teilweisen oder vollständigen Abbau der die Nukleinsäuren umgebenden Zellwände zu zerstören. Hierfür sind dem Fachmann vielfältige Verfahren bekannt, z. B. Verdau mit Hilfe von Proteasen in Gegenwart von Detergenzien oder/und chaotropen Salzen. Obgleich es prinzipiell möglich und sogar im Sinne der Erfindung bevorzugt ist, Nukleinsäuren schon aus solchermaßen hergestellten aufgeschlossenen Rohproben nachzuweisen, ist es jedoch auch möglich, die Nukleinsäuren zunächst von störenden Probenbestandteilen zu reinigen, z. B. durch Bindung der Nukleinsäuren an feste Phasen, z. B. an glasartige Oberflächen in Gegenwart von chaotropen Salzen und Entfernung nicht-gebundener Probenbestandteile. Die an die feste Phase gebundenen Nukleinsäuren können anschließend, z. B. durch Niedrigsalzpuffer, wieder von der Oberfläche entfernt werden (z. B. gemäß WO 95/01359).

Auch in der Funktion der Nukleinsäuren sind der Erfindung keine Grenzen gesetzt. Es kann sich bei den Nukleinsäuren um genomische, d. h. lange Nukleinsäuren handeln, jedoch auch um Nukleinsäuren, die schon teilweise zerkleinert sind, oder um Transkripte. Besonders bevorzugt handelt es sich bei den Nukleinsäuren um Produkte einer Nukleinsäureamplifikation, wie der Polymerasekettenreaktion (PCR), gemäß EP-B-0 201 184. In der PCR werden nachzuweisende Nukleinsäuren in der Probe dadurch vermehrt, daß die Probe mit zwei Primern in Kontakt gebracht wird, von denen einer zu einem ersten Strang und der andere zu dessen Gegenstrang der nachzuweisenden Nukleinsäure komplementär ist. Die Primer werden so gewählt, daß das Produkt einer Verlängerung des einen Primers unter Verwendung der nachzuweisenden Nukleinsäure als Matrize wiederum als Matrize zur Verlängerung des anderen Primers dienen kann. Die Verlängerung findet durch eine DNA-Polymerase mit Monodesoxyribonukleosidtriphosphaten statt. Zwischen den einzelnen Verlängerungsschritten werden die Verlängerungsprodukte von den jeweiligen Matrizen durch Denaturierung, z. B. durch einen Hitzeschritt, getrennt. Durch eine solche Amplifikation ist es möglich, spezifisch einen bestimmten Teil einer Nukleinsäure, selbst in Gegenwart anderer Nukleinsäuren, zu vermehren. Es kann sich daher bei den nachzuweisenden Nukleinsäuren nicht nur um natürlich vorkommende Nukleinsäuren, sondern auch um amplifizierte oder/und modifizierte Nukleinsäuren handeln.

Ein Charakteristikum der nachzuweisenden Nukleinsäure ist die Anwesenheit einer Teilsequenz S in der Nukleinsaure. Diese Teilsequenz besteht aus der Aufeinanderfolge von Nukleinsäurebasen einer bestimmten und vorbekannten Sequenz. Die Teilsequenz ist bevorzugt größer ais 5 Nukleotide, bevorzugt zwischen 6 und 100 Nukleotiden, besonders bevorzugt zwischen 10 und 30 Nukleotiden lang. Die Teilsequenz befindet sich bevorzugt an einem für die Sonde zugänglichen Bereich der Nukleinsäure. Sie enthält die Sequenzinformation, die Gegenstand des Nachweises sein soll, z. B. eine Sequenz, innerhalb der eine bestimmte Mutation vermutet wird oder die für einen bestimmten Organismus charakteristisch ist. Bei dieser Mutation kann es sich um eine oder mehrere Punktmutationen, um eine oder mehrere Deletionen, um eine oder mehrere Insertionen oder um Polymorphismen handeln. Besonders bevorzugt sind solche Mutationen, die ein Zeichen für eine Infektion durch einen Organismus, eine genetische Erkrankung oder eine Prädisposition für eine solche Erkrankung darstellen. Beispiele fiir solche Mutationen sind die Abweichungen im P53-Gen. Ein Beispiel fiir den Nachweis einer Infektion ist der Nachweis von Hepatitis C-Virus aufgrund der Anwesenheit von HCV-RNA, wie sie in EP-A-0 318 216 beschrieben ist. Für diesen Fall wird beispielsweise aus der HCV-RNA eine cDNA erzeugt und diese mit Hilfe der PCR in dem gewünschten Teilbereich, z. B. innerhalb der nicht-codierenden Region, amplifiziert.

Das erfindungsgemäße Verfahren eignet sich besonders zum parallelen Bestimmen von Nukleinsäuren unterschiedlicher Sequenz, Herkunft oder/und Funktion. Dies geschieht dann basierend auf unterschiedlichen Teilsequenzen dieser Nukleinsäuren. Die Teilsequenzen S können gleich lang oder verschieden lang sein, haben aber bevorzugt unterschiedliche Basensequenzen.

Unter einer Sonde P wird im Sinne der Erfindung ein Molekül verstanden, welches an die Teilsequenz S der nachzuweisenden Nukleinsäure binden kann. Die Bindung findet über Basen-Basenwechselwirkung, insbesondere Watson-Crick- oder/und Hoogsten-Basenpaarung statt. Die Sequenz der Sonde P wird bevorzugt so gewählt, daß sie im Bereich der Teilsequenz S vollständig, d. h. zu 100 % komplementär zu dieser Teilsequenz ist. Diese Bedingung ist jedoch nur dann unbedingt erforderlich, wenn sich Nukleinsäuren in der Probe befinden, die eine Teilsequenz enthalten, die der Teilsequenz S sehr ähnlich ist, die aber nicht nachgewiesen werden sollen, d. h. für den bevorzugten Fall der Verwendung einer für die nachzuweisende Nukleinsäure spezifischen Sonde. Andererseits sind mit der vorliegenden Erfindung auch gemeinsame Nachweise aller Nulcleinsäuren möglich, die eine bestimmte Teilsequenz aufweisen, sich jedoch in anderen Bereichen unterscheiden (z. B. Mitglieder einer topologischen Gruppe, z. B. Bakterien einer Familie), indem entweder die Sonde einzelne, gegebenenfalls unterschiedliche Mismatches zu den verschiedenen, als Gruppe nachzuweisenden, Nukleinsäuren aufweist, jedoch so lang ist, daß dennoch eine Hybridisierung mit allen Mitgliedern der Gruppe stattfindet, oder Hybridisierungsbedingungen für die Bindung der Sonde gewählt werden, die trotz des Auftretens von Mismatches eine Hybridisierung mit allen Mitgliedern der Gruppe erlauben, oder die Sondensequenz aus einem innerhalb der Gruppe stark konservierten Sequenzbereich ausgewählt wird.

Die Sonde P ist ein Molekül, welches die Nukleinsaure im Bereich der Teilsequenz S gegen Abbau schützt. Bevorzugt ist die Sonde auch selbst resistent gegenüber dem Abbau unter den gewählten Bedingungen. Es handelt sich daher um Sonden mit einem Grundgerüst, welches von dem natürlichen Zuckerphosphatgrundgerüst abweicht, nämlich einem Peptidgrundgerüst. Bevorzugte Sonden sind in der WO 92/20702 beschrieben. Diese Sonden haben eine, verglichen mit den entsprechenden Oligonukleotiden gleicher Basensequenz, erhöhte Affinität zu komplementären Nukleinsäuren. Sie können insbesondere in DNA-DNA-Doppelstränge eindringen.

Die Sonde P im Sinne der Erfindung hat zweckmäßigerweise eine definierte Länge und Zusammensetzung, insbesondere eine definierte Größe oder/und Masse. Insbesondere ist die Masse der Sonde P im voraus bekannt.

Aus der nachzuweisenden Nukleinsäure und der Sonde P wird in einem ersten Schritt ein Bindeprodukt B1 gebildet. Hierzu wird die nachzuweisende Nukleinsäure enthaltende Probe mit der Sonde P in Kontakt gebracht. Die Sonde P befindet sich bevorzugt in Lösung und wird mit einer bestimmten Menge Probenflüssigkeit vereinigt. Das Bindeprodukt kann doppelsträngig sein, d. h. es ist jeweils ein Strang der Sonde P an einen Strang der nachzuweisenden Nukleinsäure gebunden, oder dreifachsträngig sein, z. B. indem zwei Stränge der nachzuweisenden Nukleinsäure an einen Strang der Sonde oder zwei Sonden oder zwei Teile einer Sonde an einen Strang der Nukleinsäure binden. Die Bedingungen für die Bindungen richten sich nach der Art der Sonde und der Nukleinsäure. Im Falle einer Hybridisierung kann z. B. die Stringenz durch Variation der Salzkonzentration, der Temperatur etc. eingestellt werden. Auch die Spezifität der Bindung kann mit diesen Parametern beeinflußt werden. Für den Fall der Verwendung von Peptidnukleinsäuren (PNA) der WO 92/20702 ist es auch möglich, bei sehr niedrigen Salzkonzentrationen zu arbeiten (kleiner als 100 µM). Die Bindung der Sonde an die Nukleinsäure wird im einfachen Fall durch Zugabe der Sonde zu der die Nukleinsäure enthaltenden Probenlösung eingeleitet. Die Sonde kann hierbei in geloster oder fester Form, als Einzelreagenz oder als Reagenzgemisch mit weiteren, für die Bindung oder weitere Schritte erforderlichen oder gewünschten Reagenzien, geschehen. Weitere Reagenzien können beispielsweise Puffer sein. Prinzipiell ist jedoch jede Art der Vereinigung der Sonde P mit der Nukleinsäuren enthaltende Lösung möglich, z. B. Zugabe der Probe zu einer Lösung der Sonde.

In einem darauffolgenden Schritt wird die Probe mit dem Bindeprodukt B1 Bedingungen unterworfen, bei denen die Nukleinsäure unter Erzeugung eines Bindeprodukts B2 abgebaut wird. Wesentlich im Sinne der Erfindung ist es, daß durch den Abbau eine Teilnukleinsäure F definierter Länge erzeugt wird. Gelegentlich kann es vorkommen, daß der Abbau ein Gemisch von Teilnukleinsäuren erzeugt. Dies kann der Fall sein, wenn der Abbau relativ lange durchgeführt wird. Dann können sich auch Produkte bilden, die von den Enden her um einige Nukleotide, bevorzugt weniger als 5 Nukleotide, kürzer sind als die Sonde P. Bevorzugt enthält die Teilnukleinsäure F demnach einen zu der Teilsequenz S komplementären Teil, wenngleich auch schon die Tatsache der Hybridisierung der Sonde P und somit die Bildung einer Teilnukleinsäure F als Indiz für die ursprüngliche Anwesenheit der Teilsequenz S dienen kann. Dies ist nicht störend, solange das Hauptprodukt, welches Gegenstand der späteren Messung werden soll, meter als ca. 50 %, bevorzugt meter als 70 %, des Gemisches ausmacht. Die Empfindlichkeit der Bestimmung ist natürlich umso höher, je meter Hauptprodukt vorliegt. Welches das Hauptprodukt des Abbaus ist, d. h. welche Teilnukleinsäure zum Gegenstand der Massenbestimmung gemacht werden soll, kann der Fachmann auch vorab durch Aufnahme eines Massenspektrums und Selektion des höchsten Signals erkennen. Diese Teilnukleinsäure ist Teil der ursprünglich an die Sonde P gebundenen Nukleinsäure. Die Teilsequenz S wird hierbei nicht abgebaut. Wenngleich es auch möglich ist, den Abbau der Nukleinsäure nicht vollständig bis auf die Teilsequenz S durchzuführen, ist der Fall des vollständigen Abbaus der Nukleinsäure unter Zurücklassung nur der Basen, an die die Sonde P bindet, bevorzugt. Unter Abbau der Nukleinsäure wird bevorzugt die Verringerung der Länge der Nukleinsäure, insbesondere in Bereichen außerhalb der Teilsequenz S, z. B. unter Entfernung von einzelsträngigen Bereichen, einer oder mehrerer Doppelstrangspaltungen, aber insbesondere unter Erhaltung der Bindung der Sonde P an die Teilsequenz S, verstanden.

Der Abbau kann auf prinzipiell jede Weise geschehen Bevorzugt geschieht er jedoch enzymatisch. Geeignete Enzyme haben Einzelstrang- und/oder Doppelstrang-verdauende sowie DNA- und/oder RNA-verdauende Aktivitäten, wie Nukleasen, insbesondere DNasen, RNasen oder Restriktionsendonukleasen, z. B. DNase I aus Rinderpankreas (z. B. Lindberg, U., Biochemisty, 1967, 6, 335, Lankowski, M., The Enzymes, 1961, 5, 125; Clark, P. et al., Biochemistry 1974, 13, 5098), Exonuklease V aus Micrococcus luteuas (z. B. Anal, M. et al., J. Biol. Chem. 1970, 245, 767), Nuklease S7 aus Staphylococcus aureus (z. B. Alexander, M et al., J. Biol. Chem., 1961, 236, 3014; Chsaka, A. et al., J. Biol. Chem., 1964, 239, 3498; Lankowski, M., The Enzymes, 1961, 5, 142), Nuklease S1 aus Aspergillus oryzae (z. B. Ando, T., Biochim. Biophys. Acta, 1966, 114, 158; Vogt, V.M., Eur. Biochem., 1973, 33, 192; Vogt, V.M., Methods Enzymol. 1980, 65, 248) oder Mung Bean Nuklease aus den Sprößlingen der Mungobohnen (z. B. Sung, S.C. et al., J. Biol. Chem, 1962, 237, 506). Das Enzym wird je nach Art der abzubauenden Nukleinsäure (ds DNA oder/und RNA) ausgewählt. Auch der Einsatz von Enzymmischungen, z. B. DNase I und Nuklease S1, ist vorteilhaft.

Die Abbaureaktion wird bevorzugt so lange fortgesetzt, bis die an die Sonde gebundenen Nukleinsäuren eine definierte, konstante Länge aufweisen. Bevorzugt sollen auch alle, neben der nachzuweisenden Nukleinsäure in der Probe befindlichen weiteren, nicht nachzuweisenden Nukleinsäuren, möglichst vollständig abgebaut sein. Dieser Vorgang ist von den verwendeten Bedingungen abhängig, wird jedoch in der Regel in weniger als 2 Stunden, bevorzugt weniger als 30 Minuten abgeschlossen sein. In der Lösung befinden sich dann das Bindeprodukt B2 rnit einer definierten mittleren Masse (abhängig insbesondere von der Länge der Sonde P) sowie gegebenenfalls überschüssige Sonde P mit einer niedrigeren, aber definierten Masse, niedermolekulare Abbauprodukte, z. B. Mononukleotide mit meist undefinierten und unterschiedlichen niedrigen Massen und Enzyme, z. B. DNase rnit hohen Massen (in der Regel mehr als 20000 D). Das Bindeprodukt B2 setzt sich nun zusammen aus der Sonde P und der verkürzten Nukleinsäure (Teilnukleinsäure F), die die Teilsequenz S enthält. Das Bindeprodukt B2 hat daher eine Masse, die bei konstanter, definierter Sondenmasse SM von der Länge und Basenzusammensetzung der Teilsequenz F mit der Masse FM abhängt. Durch Wahl der Abbaubedingungen kann nun die Masse M des Komplexes zur Bestimmung entweder der Länge oder der Basenzusammensetzung genutzt werden. Die Basenzusammensetzung ist darüber hinaus abhängig von der Sequenz der nachzuweisenden Nukleinsäure innerhalb der Teilsequenz S. Gewünschtenfalls kann auch nach dem Abbau und vor der Messung eine Aufreinigung der Bindeprodukte oder Teilen davon durchgeführt werden, wenngleich die Erfindung gerade für die Messung im Gemisch vorteilhaft ist.

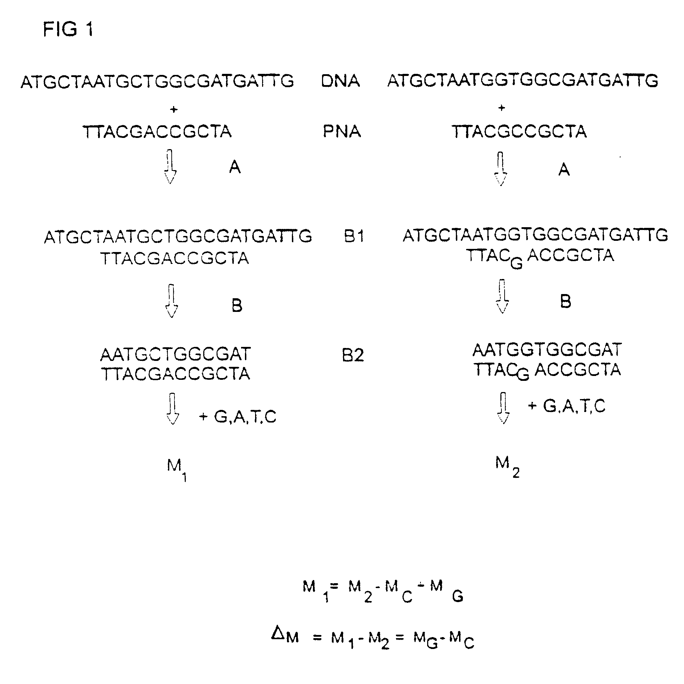

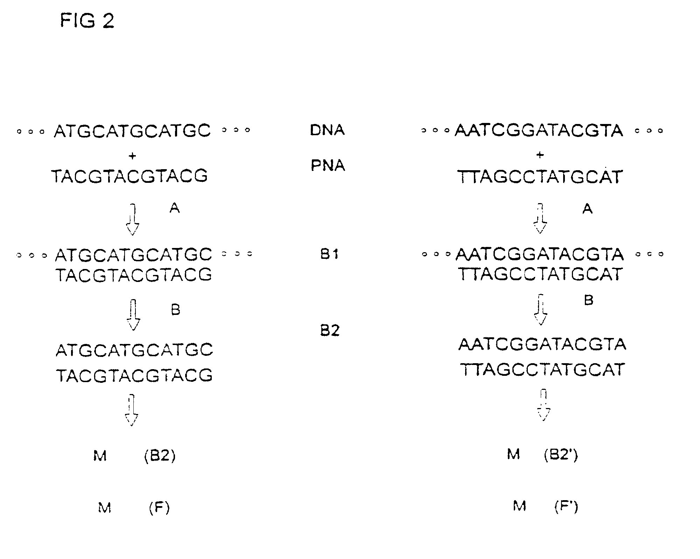

Die Möglichkeiten, die sich durch die Wahl der Abbaubedingungen für den Nachweis einer Nukleinsäure ergeben, sind in Figur 1 und Figur 2 gezeigt. In Figur 1 ist das erfindungsgemäße Verfahren bei Vorliegen zweier Nukleinsäuren (DNA) gezeigt, die sich in einer Position (Position 8) in der Basensequenz unterscheiden. Es ist gezeigt, daß Bindung der PNA-Sonde an DNA (Reaktion A) und anschließender Verdau der überstehenden einzelsträngigen Teile (Reaktion B) zu zwei definierten Komplexen mit jeweils unterschiedlichem Molekulargewicht M1 bzw. M2 führen. Die beiden Komplexe können anhand ihres Unterschiedes im Molekulargewicht, der genau der Differenz zwischen den Basen C und G entspricht, eindeutig zugeordnet und identifiziert werden.

In Figur 2 ist der Fall gezeigt, daß zwei verschiedene Nukleinsäuren simultan bestimmt werden können. Die beiden Nukleinsäuresequenzen haben jeweils verschiedene Teilsequenzen S. Es werden unterschiedlich lange, zur jeweiligen Teilsequenz S komplementäre PNA-Proben (12-mer und 13-mer) an die entsprechenden Nukleinsäuren anhybridisiert. Es bilden sich die Bindeprodukte B1 und B1'. Nach enzymatischem Verdau der nicht durch die Proben geschützten Teilsequenzen S erhält man die Bindeprodukte B2 und B2' mit unterschiedlichen Massen M1 (B2) und M2 (B2). Die gezeigten Reaktionen können gleichzeitig in einem einzigen Ansatz durchgeführt werden. Durch die Anwesenheit des Bindesproduktes B2 kann auf die Anwesenheit der nachzuweisenden Nukleinsäure geschlossen werden. Hierzu wird die Masse entweder des Bindeprodukts B2 oder eines Teils hiervon bestimmt. Telle von B2, die sich für eine Bestimmung eignen, sind z. B. die Sonde P und die Teilnukleinsäure F. Bevorzugt ist die Bestimmung der Teilnukleinsäure F. Dies kann geschehen, indem z. B. das Bindeprodukt B2 unter Freisetzung der Teilnukleinsäure zerstört wird, z. B. durch Strangtrennung, wie bei Behandlung mit Alkali.

In alternativen Verfahren kann die Bestimmting jeweils auch über die Detektion der Masse, eines Teils des Bindeproduktes B2, insbesondere eines Teils mit mehr als 5 Basen, bevorzugt der Teilnukleinsaure F, die bei der Ionisierung aus dem Bindeprodukt B2 freigesetzt wird, erfolgen.

Der Nachweis des Bindeprodukts B2 findet im Sinne der Erfindung aufgrund seiner Masse start. Die Masse von ein- oder doppelsträngiger Nukleinsäure kann prinzipiell massenspektrometrisch bestimmt werden. Zur Aufnahme eines Massenspektrums des Bindeproduktes B2 der Teilnukleinsäure F oder der Sonde P eignen sich gemäß dem heutigen Stand der Technik "weiche" Ionisationsverfahren, besonders geeignet ist das Verfahren der "atmospheric pressure ionization" (API), insbesondere "electrospray ionization" (ESI, gemäß Griffith et al., JACS 1995, 117, 831). In Analytical Chemistry, Vol 68, 3, 527-533 (1996) ist ein ebenfalls geeignetes Verfahren beschrieben. Relevant für das hier beschriebene Nachweisverfahren ist lediglich das verwendete Ionisationsverfahren. Zur Massenbestimmung der bei der Ionisation gebildeten Ionen können grundsätzlich alle mit diesem Ionisationsverfanren koppelbaren Massenanalyser verwendet werden, z. B. Quadrupolmassenspektrometer, Flugzeitmassenspektrometer, Ionenfallen oder Sektorfeldgeräte. Die Massenanalyser können zur Verbesserung der Selektivität oder Empfindlichkeit auch im MS/MS oder MSº Modus betrieben werden. Als Ionisationsverfahren kann auch die Matrix unterstützte Laserdesorptions-Ionisation (MALDI) angewendet werden. Besonders geeignet erscheint das MALDITOF-Verfahren nach U. Pieles et al., Nucl. Acids Res. 1993, Vol. 21, 3191 oder Anal. Chem. 1995, 67, 4139-4144). Die Messung kann sowohl mit positiv geladenen Ionen, als auch bevorzugt mit negativ geladenen Ionen erfolgen. Zur Messung der Probe mit negativ geladenen Ionen mit ESI wird das Reaktionsgemisch zum Beispiel in Ammoniumacetat-Puffer gelöst und diese Pufferlösung dem Ionisationssystem mit Flußraten zwischen 10 nl/min und 1 ml/min zugeführt. Die Bildung der Ionen erfolgt durch Zerstäuben der Lösung in einem elektrischen Feld, wobei der Prozeß pneumatisch durch Zufuhr eines Gases oder thermisch unterstützt werden kann. Die gebildeten Ionen werden im elektrischen Feld fokussiert und über geeignete Blenden oder Kapillaren in den Hochvakuumbereich des Massenspektrometers überführt. Unter diesen Ionisationsbedingungen entstehen typischerweise Ionenserien von einfach und mehrfach deprotonierten Motekülen, deren Verhältnis Masse zu Ladung mit z. B. einem Quadrupol-Massenanalyser bestimmt wird. Aus den erhaltenen Ionenserien läßt sich das Molekulargewicht der in der Probe enthaltenen Komponenten berechnen (J. B. Fenn et al., Science 1989, 246, 64 - 70). Die Intensitäten der erhaltenen Ionen korrelieren mit der Konzentration der Komponenten in der Lösung. Beim Ionisationsprozeß zum Teil auftretende Addukte, wie z. B. Natrium oder Kalium-Addukte, können ebenfalls zur Auswertung verwendet werden. Die Messung von positiv geladenen Ionen erfolgt in z. B. Essigsäure-haltigen Pufferlösungen, wobei bei der Ionisation Serien von protonierten Molekülen erzeugt werden. Die Messung und Auswertung erfolgt analog der Messung im Negativmodus.

Geräte zur Aufnahme solcher Massensprekten sind beispielsweise von den Firmen Finnigan MAT, Perkin Elmer Sciex, Bruker, MicroMass und anderen erhältlich. Die Genauigkeit der Messung sollte bei ca. 0.01 % (ESI) bzw. 0,1 % (MALDITOF) Abweichung von der errechneten Masse liegen, was ca. ± 1 - 2 Dalton entspricht.

Alternativ zur Massenbestimmung der Probe direkt in dem kompletten Reaktionsgemisch kann vor der Messung eine Probenaufreinigung oder Anreicherung durchgeführt werden. Dazu kommen bevorzugt Filtrationsprozesse (Ultrafiltration) oder chromatographsiche Reinigungsverfahren zum Einsatz, wobei insbesondere die chromatographischen Verfahren automatisierbar sind und als direkte Kopplungsverfahren mit der Massenspektrometrie verwendet werden können. Chromatographsiche Verfahren sind z. B. Reversed Phase-, Ionenaustauscher-, Gelpermeations- oder Affinitätschromatorgraphie. Gut geeignet sind auch Elektrophorese, insbesondere Kapillarelektrophoreseverfahren. Die Masse des Bindeprodukts B2 ist direkt oder indirekt indikativ für die nachzuweisende Nukleinsäure. Für den Fall der linken Spaite in Figur 1 kann die erwartete Masse direkt aus der verwendeten Sondensequenz ermittelt werden, da sich aus der 100%igen Komplementarität der Sonde zu der Teilsequenz S ein definiertes Molekulargewicht ergibt. Das Erscheinen eines Signals für genau diese Masse ist daher als Zeichen für die Anwesenheit der nachzuweisenden Nukleinsäure indikativ. Auch die in anderen Verfahren detektierte Masse der Teilnukleinsäure F ist ebenso indikativ für die Anwesenheit der nachzuweisenden Nukleinsäure.

Im Fall der rechten Spalte von Figur 1 kann aus der Abweichung ΔM geschlossen werden, daß sich die nachzuweisende Nukleinsäure von der im Fall 100%iger Komplementarität zu erwartenden Sequenz um den Austausch C/G unterscheidet. Dies kann dazu herangezogen werden, genau diese Mutation nachzuweisen. Die Mutation C->G liegt vor, wenn sich das bestimmte Signal in seiner Masse um die Differenz der Basen G und C von dem Signal unterscheidet, weiches für 100%ige Komplementaritat errechnet wurde. Dies zeigt, daß das erfindungsgemäße Verfahren für die spezifische Bestimmung von einzelnen Mutationen ganz vorteilhaft herangezogen werden kann, da die Größe der Differenz zwischen den gemessenen und dem erwarteten Signal ein Anzeichen für einen Basenaustausch, insbesondere einen ganz bestimmten, ist.

Das erfindungsgemäße Verfahren kann jedoch auch zum simultanen Nachweis von Nukleinsäuren unterschiedlicher Sequenz eingesetzt werden, wie in Figur 2 gezeigt. Hierzu werden anstelle einer Sonde P zwei oder mehr Sonden P1, P2 ... PN mit der Probe in Kontakt gebracht. Für jede dieser Sonden ergibt sich bei Vorhandensein der Nukleinsäure ein Signal bei der vorbestimmten Masse. Da die Anzahl und Auswahl möglicher Sonden P mit unterschiedlichen Sequenzen und gegebenenfalls unterschiedlicher Lange praktisch unbegrenzt ist, kann dieses Verfahren zum Nachweis von praktisch beliebig vielen unterschiedlichen Nukleinsäuren herangezogen werden. Gerade in diesem Fall ist jedoch bei den Verfahren des Standes der Technik eine Vielzahl von unterschiedlichen Markierungen und gegebenenfalls unterschiedlichen Detektionsverfahren erforderlich. Ebenfalls Gegenstand der Erfindung ist daher ein Gemisch von Sonden P1, P2 ... PN unterschiedlicher Masse, wobei diese Sonden löslich sind in üblichen nukleinsäurehaltigen Lösungen und wobei N eine natürliche Zahl ist und die Zahl unterschiedlicher Sonden angibt. Die unterschiedliche Masse wird entweder durch unterschiedliche Sequenzen oder durch unterschiedliche Länge oder durch unterschiedliche Modifizierungsgruppen oder Kombinationen davon erreicht. Die übrigen erfindungsgemäßen Eigenschaften der Sonden gelten auch hier. So ist jede der Sonden P1, P2 ... PN zur Bildung eines unterschiedlichen Bindeprodukts B1 mit der hierzu passenden Nukleinsäure, sofern sie in der zu untersuchenden Probe enthalten ist, in der Lage. Die Sonden sind solche, die nicht natürlich vorkommen, nämlich PNA. Die verschiedenen Sonden sind bevorzugt in jeweils gleichen oder zumindest nicht stark unterschiedlichen Mengenanteilen vorhanden, um bei der Bestimmung ungefähr gleiche Bedingungen verwenden zu können.

Das erfindungsgemäße Verfahren eignet sich auch besonders zum Nachweis von Mutationen, wenn jeweils eine Sonde eingesetzt wird, die zu 100 % komplementär zu dem Bereich der Allele ist, in dem sich die Mutation befinden könnte.

Das erfindungsgemäße Verfahren eignet sich auch besonders zum quantitativen Nachweis von Nukleinsäuren, wenn der Reaktionsmischung Nukleinsäuren oder Fragmente bekannter Sequenz (Standardnukleinsäuren SN) und hierzu passenden (z. B. 100 % komplementären) Standardsonden SP zugesetzt werden und diese derselben Behandlung wie die nachzuweisenden Nukleinsäuren unterzogen werden. In diesem Fall wird bei einer vorbestimmten Masse ein Signal erwartet und auch gemessen werden können. Die Intensität des Signals ist proportional der Konzentration Die Höhe des Signals für die (bekannte oder definierte Menge) Standardnukleinsäure kann nun zur Bestimmung der Menge an nachzuweisender Nukleinsäure verwendet werden, indem die Höhe des Signals bei der für die nachzuweisende Nukleinsäure erwarteten Masse mit der Höhe des Signals für die Standardnukleinsäure verglichen oder/und ins Verhältnis gesetzt wird.

Neben einer quantitativen Auswertung erlaubt die Verwendung einer Standardnukleinsäure aber auch eine Kontrolle für das ordnungsgemäße Funktionieren des Tests. Sollte kein Signal gemessen werden können, ist der Test fehlerhaft.

In einer Variante kann die Sonde P auch markiert sein, z. B. durch eine chemische Gruppe, die eine Sonde mit einer spezifischen Sequenz von einer Sonde derselben Masse, jedoch anderer Sequenz unterscheidet. Dies ermöglicht auch die Unterscheidung von Nukleinsäuren mit sehr nahe verwandten Sequenzen. Bei simultanen Nachweisen können auch mehrere unterschiedlich markierte Sonden verwendet werden. Die Sonden können auch mit Gruppen versehen sein, die die Ionisierung erleichtern, z. B. negativ geladene Gruppen für die Aufnahme eines Massenspektrums im "negativ-Mode", wie Carboxyl- oder Sulfonsäuregruppen, oder positiv geladene Gruppen für den "positiv-Mode". Diese Gruppen sind bevorzugt mehrfach vorhanden, d. h. die Sonde verhält sich dann poly- oder oligo-an- oder kationisch. Diese Gruppen erhöhen die Sensitivität der massenspektrometrischen Bestimmung.

Aus dem erfindungsgemäßen Verfahren kann die Anwesenheit einer Teilsequenz S auf zwei Stufen ermittelt werden. Zum einen ist schon die Anwesenheit, d. h. das Überstehen der Abbaubedingungen, einer definierten Teilnukleinsäure F ein Indiz für das Vorliegen der Teilsequenz S, zum andern kann die Bestimmung der Masse entweder zur Bestätigung der Sequenz der Teilnukleinsäure F bzw. der Teilsequenz S oder zur Ermittlung von Abweichungen in der Sequenz dienen.

Das erfindungsgemäße Verfahren kann auf vielerlei Weise erfolgreich genutzt werden. Es handelt sich optional um ein homogenes Verfahren, d. h. die beteiligten chemischen Reaktionen finden bevorzugt in einer Phase, bevorzugt der flüssigen Phase, statt. Solche Reaktionen laufen unter besser kontrollierbaren Bedingungen und schneller ab. Darüber hinaus kann das erfindungsgemäße Verfahren schneller sein als die bisher verfügbaren Verfahren. Es kann auch sensitiver sein als herkömmliche Verfahren und unter Umständen kann sogar eine Amplifikation der nachzuweisenden Nukleinsauren vermieden werden. Der Einsatz markierter Sonden kann vermieden werden. Da das Vorhandensein einer ganz bestimmten Masse für das Testergebnis herangezogen wird, kann apparativ für eine hohe Auflösung genau in dem zur Rede stehenden Massenbereich gesorgt werden. Die übrigen, während des Verfahrens anfallenden oder eingesetzte Produkte (z. B. einzelsträngige Fragmente oder Sonden) haben eine von dem Bindeprodukt B2 oder von der Teilnukleinsäure F deutlich unterschiedliche Masse.

Das Verfahren ist auch automatisierbar. Darüber hinaus eignet sich das erfindungsgemäße Verfahren hervorragend zur simultanen Bestimmung mehrerer Nukleinsäuren mit unterschiedlichen Sequenzen oder unterschiedlicher Sequenzen einer Nukleinsäure. Hierzu wird dann eine Kombination mehrerer PNAs eingesetzt, die zu diesen Sequenzen komplementär sind.

In einer besonders bevorzugten Ausführungsform läuft das Verfahren folgendermaßen ab:

In einem ersten Schritt wird die nachzuweisende Nukleinsäure gegebenenfalls durch Lyse aus Zellen freigesetzt. Die Nukleinsäure wird anschließend an einer festen Oberfläche, z. B. einer glasartigen Oberfläche, immobilisiert und die übrige Probenflüssigkeit entfernt. Dann wird die Nukleinsäure von der Oberfläche abgelöst und eine Teilsequenz durch PCR amplifiziert.

In einem nächsten Schritt wird an eine Teilsequenz S einer amplifizierten Nukleinsäure eine PNA-Probe P mit komplementärer Sequenz zu einem Bindeprodukt B1 anhybridisiert. In einem weiteren Schritt wird das Bindeprodukt B1 mit einer Nukleasemischung in einem für die verwendeten Enzyme optimalen Enrympuffer und bei optimaler Temperatur zum Bindeprodukt B2, das nun aus der Teilnukleinsäure F mit der Teilsequenz S und der PNA-Probe P besteht, digestiert. B2 wird mittlel S Ionenaustausch-Chromatographie von anderen Reaktionskomponenten befreit und anschließend die B2-haltige Fraktion dem massenspektrometrischen System zugefuhrt. Die Masse des Bindeprodukts B2 bzw. der Teilnukleinsäure F wird bestimmt.

Das folgende Beispiel soll die Erfindung näher erläutern:

### Beispiel

Es werden 2.7 nmol (5 µl in Wasser) Oligonukleotid der Sequenz 5'-TAT TAG GCC ATC GTC TTC ATG GTC CAG AAC ACC AAC AGG AAG ACC-3' (SEQ.ID.NO. 1) und 2.7 nmol (5 µl in Wasser) PNA der Sequenz H₂N- AAG TAC CAG GTC TTG-H zusammenpipettiert, dann gibt man 10 µl 2x Assay Puffer (60 mM Na-acetat, 100 mM NaCl, 2 mM Znacetat, 0.002% Triton X-100, pH 4.6) zu. Die Probe wird auf 95°C für 3 min. erwärmt, dann auf 37°C abgekühlt. Darauf gibt man 1 U Mung Bean Nuclease I (Boehringer Mannheim) zu und inkubiert die Probe 10 min bei 37°C. Die Enzymreaktion wird durch Zugabe von 1 mM EDTA (25 µl) gestoppt. Danach wird die Lösung mit Wasser auf 0.5 ml verdünnt und über NAP-5 (Pharmacia) entsalzt.

Für die massenspektrometrische Analyse wurde ein Verfahrn entsprechend Wilm et al., Analytical Chemistry 68 (1996), 527-533 (Parent Ion Scans of Unseparated Peptide Mixtures) verwendet. Massenspektrometer API III triple quadrupol mit collision cell (Perkin-Elmer Sciex Instruments) mit Nonoelektrospray Ionenquelle; Polarität negativ Mode, Spray Potential - 750 V, Scan Range 350-1400 Da, Step Width 0.2 Da, Dwell Time 1 ms; 11 scans added up, Precursor ion scan mode für Ion m/z 79, Collision gas Argon, Collision energy 35 eV/charge.

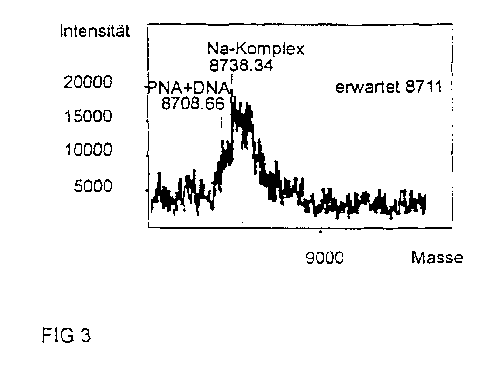

Ein typisches Spektrum für dien PNA-DNA Duplex ist in FIG. 3 dargestellt. Nach Deconvolution der Rohdaten erhält man für den Duplex ein Molekulargewicht von 8709 Da (theoretisch erwartet 8711 Da).

## Patentansprüche

1. Verfahren zum Nachweis einer Nukleinsäure enthaltend die Schritte
- Bindung einer Sonde P an eine in der Nukleinsäure enthaltenen Teilsequenz S unter Erzeugung eines Bindeproduktes B1, **dadurch gekennzeichnet, daß** die Sonde P ausgewahlt ist aus der Gruppe der Peptidnukleinsäuren
- Abbau der Nukleinsäure unter Erzeugung eines Bindeproduktes B2, enthaltend eine Teilnukleinsäure F definierter Länge unter Erhalt der Bindung an die Sonde P.
- Nachweis des Bindeprodukts B2, **dadurch gekennzeichnet, daß** der Nachweis des Bindeproduktes massenspektrometrisch erfolgt.

2. Verfahren gemäß Anspruch 1, **dadurch gekennzeichnet, daß** die Sonde P in der Lage ist, den Abbau der Nukleinsäure im Bereich der Teilsequenz S zu verhindern.

3. Verfahren gemäß Anspruch 1 oder 2, **dadurch gekennzeichnet, daß** der Abbau enzymatisch geschieht.

4. Verfahren gemaß Anspruch 3, **dadurch gekennzeichnet, daß** das Enzym oder eine Enzymmischung eine Einzelstrang- und/oder Doppelstrang-verdauende sowie DNAund/oder RNA-verdauende Aktivität hat.

5. Verfahren gemäß Anspruch 3, **dadurch gekennzeichnet, daß** das Enzym ausgewählt ist aus der Gruppe S1-Nuklease, DNase I, Micrococcus Nuklease und Mung bean Nuklease.

6. Verfahren gemäß Anspruch 1, **dadurch gekennzeichnet, daß** mehrere Nukleinsäuren simultan nachgewiesen werden, indem an jede der nachzuweisenden Nukleinsäuren eine Sonde unterschiedlicher Masse gebunden und diese Bindeprodukte den in Anspruch 1 genannten Schnitten unterworfen werden.

7. Verfahren gemäß Anspruch 1, **dadurch gekennzeichnet, daß** die Nukleinsäuren vor der Bindung der Sonde vorgereinigt werden.

8. Verfahren gemäß Anspruch 1, **dadurch gekennzeichnet, daß** die masenspektrometrische Auswertung quantitativ erfolgt.

9. Reagenz zum simultanen massenspektrometrischen Nachweis einer Vielzahl von Nukleinsäuren unterschiedlicher Sequenz, enthaltend ein Gemisch einer Vielzahl von Peptidnukleinsäuresonden unterschiedlicher Masse in löslicher Form.

## Claims

1. Process for the detection of a nucleic acid, comprising the steps
- bonding of a probe P to a part sequence S contained in the nucleic acid to produce a bonding product B1, **characterized in that** the probe P is chosen from the group consisting of peptide nucleic acids
- breakdown of the nucleic acid to produce a bonding product B2 containing a part nucleic acid F of defined length, the bond to the probe P being preserved
- detection of the bonding product B2, **characterized in that** the detection of the bonding product is carned out by mass spectrometry.

2. Process according to claim 1, **characterized in that** the probe P is capable of preventing the breakdown of the nucleic acid in the region of the part sequence S.

3. Process according to claim 1 or 2, **characterized in that** the breakdown takes place enzymatically.

4. Process according to claim 3, **characterized in that** the enzyme or an enzyme mixture has a single strand- and/or double strand-digesting and DNA- and/or RNA-digesting activity.

5. Process according to claim 3, **characterized in that** the enzyme is chosen from the group consisting of S1 nuclease, DNase I, Micrococcus nuclease and mung bean nuclease.

6. Process according to claim 1, **characterized in that** several nucleic acids are detected simultaneously, **in that** a probe of different mass is bonded to each of the nucleic acids to be detected and these bonding products are subjected to the steps mentioned in claim 1.

7. Process according to claim 1, **characterized in that** the nucleic acids are prepurified before the bonding of the probe.

8. Process according to claim 1, **characterized in that** the evaluation by mass spectrometry is carried out quantitatively.

9. Reagent for simultaneous detection of a large number of nucleic acids of different sequence by mass spectrometry, comprising a mixture of a large number of peptide nucleic acid probes of different mass in soluble form.

## Revendications

1. Procédé de mise en évidence d'un acide nucléique, comprenant les étapes suivantes
- la liaison d'une sonde P à l'une des séquences partielles S contenues dans l'acide nucléique avec production d'un produit de liaison B1, **caractérisée en ce que** la sonde P est choisie dans le groupe des acides nucléiques peptidiques,
- la dégradation de l'acide nucléique avec production d'un produit de liaison B2, contenant un acide nucléique partiel F de longueur définie avec maintien de la liaison à la sonde P,
- la mise en évidence du produit de liaison B2, **caractérisée en ce que** la mise en évidence du produit de liaison s'effectue par spectrométrie de masse.

2. Procédé selon la revendication 1, **caractérisé en ce que** la sonde P est en situation d'empêcher la dégradation de l'acide nucléique dans le domaine de la séquence partielle S.

3. Procédé selon la revendication 1 ou 2, **caractérisé en ce que** la dégradation s'effectue par voie enzymatique.

4. Procédé selon la revendication 3, **caractérisé en ce que** l'enzyme ou un mélange d'enzymes a une activité de digestion d'un brin et/ou de deux brins ainsi que de l'ADN et/ou de l'ARN.

5. Procédé selon la revendication 3, **caractérisé en ce que** l'enzyme est choisie dans le groupe constitué par la nucléase S1, l'ADNase I, la nucléase de micrococcus et la nucléase de haricot mungo.

6. Procédé selon la revendication 1, **caractérisé en ce qu'**on met en évidence simultanément plusieurs acides nucléiques en liant une sonde de masse différente à chacun des acides nucléiques à mettre en évidence et en soumettant ces produits de liaison aux étapes mentionnées dans la revendication 1.

7. Procédé selon la revendication 1, **caractérisé en ce qu'**on procède à une purification préalable des acides nucléiques avant la liaison de la sonde.

8. Procédé selon la revendication 1, **caractérisé en ce que** l'évaluation s'effectue quantitativement par spectrométrie de masse.

9. Réactif de mise en évidence simultanée par spectrométrie de masse d'un grand nombre d'acides nucléiques de séquences différentes, contenant un mélange d'un grand nombre de sondes d'acides nucléiques peptidiques de masses différentes sous forme soluble.
